# FASCICULE DE BREVET EUROPEEN

(11) **EP 0 830 129 B1**
(45) Date de publication et mention de la délivrance du brevet: **20.03.2002**
(21) Numéro de dépôt: 96913593.8
(22) Date de dépôt: 15.04.1996
(51) Int. Cl.: A61K 9/20

(54) **COMPOSITIONS SOLIDES CONTENANT DU POLYETHYLENEOXYDE ET UN PRINCIPE ACTIF NON AMORPHE**
FESTE ZUSAMMENSETZUNGEN MIT EINEM NICHT-AMORPHEM WIRKSTOFF UND POLYÄTHYLENOXYD
SOLID COMPOSITIONS CONTAINING POLYETHYLENEOXIDE AND A NON-AMORPHOUS ACTIVE PRINCIPLE

(30) Priorité: 14.04.1995 FR 9504520
(43) Date de publication de la demande: 25.03.1998
(73) Titulaire: PHARMA PASS, 67400 Illkirch Graffenstaden (FR)
(72) Inventeur: SETH, Pawan, F-67000 Strasbourg (FR); STAMM, André, F-67870 Griesheim (FR)
(74) Mandataire: Pochart, François
(86) Numéro de dépôt international: FR9600574
(87) Numéro de publication internationale: WO9632097

(56) Documents cités:
- EP-A- 0 232 877
- US-A- 5 273 758
- BIOMATERIALS, vol. 14, no. 2, 1993, GUILDFORD, SURREY, GB, pages 83-90, XP000335514 A. APICELLA; ET AL.: "Poly(ethylene oxide) (PEO) and different molecular weight PEO blends monolithic devices for drug release"

## Description

La présente invention a pour objet de nouvelles compositions solides, notamment pharmaceutiques, contenant du polyéthylèneoxyde et un principe actif, ainsi que leur procédé de préparation.

Certains médicaments doivent être formulés sous des formes de dosage dites "retard", ou à libération prolongée.

Par ailleurs, on connaît le polyéthylèneoxyde, désigné ci-après PEO, comme un composant de comprimé destiné à une administration par voie orale. Ce composé est commercialisé par la société Union Carbide Corp. sous la dénomination Polyox®. L'utilisation de PEO pour la formulation de médicaments a par ailleurs fait l'objet de nombreux brevets.

EP-A-0 277 092, au nom de Ciba-Geigy, a pour objet une composition comprenant une enveloppe en un matériau poreux à l'eau mais non aux substances actives, enveloppant un noyau constitué d'un mélange d'une substance faiblement soluble dans l'eau, et d'un matériau hydrophile gonflant, ledit matériau consistant en un mélange de PEO et d'un polymère vinylpyrrolidone/vinylacétate. La composition objet de ce brevet est un exemple des compositions actuelles où un noyau qui s'expanse dans l'eau est entouré d'un matériau poreux à l'eau, la libération du principe actif étant retardée du fait du temps nécessaire pour l'expansion du noyau et la diffusion au travers de l'enrobage, suite à la pénétration de l'eau.

L'abrégé des brevets US-P-4 404 183 et US-P-4 343 789 décrit deux modes de réalisation d'une composition à libération prolongée. Selon le premier mode de réalisation, la composition contient du PEO, le principe actif sous forme amorphe, et un composant basique. Selon le second mode de réalisation, le principe actif est la nicardipine sous forme amorphe, le composé basique pouvant être omis.

En fait, les compositions selon l'art antérieur sont complexes, nécessitent des principes actifs spécifiques ou sous une forme spécifique. Par ailleurs, les résultats atteints ne sont pas très bons.

La présente invention fournit une composition simple, ayant vocation à s'appliquer à une multitude de principes actifs, et qui fournit un effet retard remarquable.

Ainsi, l'invention a pour objet une composition solide telle que définie dans la revendication 1.

Le terme "composition solide" signifie que la composition est sous forme de comprimé ou de mini-comprimés, ceux-ci pouvant à leur tour être placés dans une gélule, par exemple classiquement en gélatine dure.

Le terme "principe actif" doit être compris dans son sens commun, et recouvre de façon générale les médicaments pour le traitement du corps humain ou animal, ainsi qu'une association d'un ou plusieurs de ces médicaments. Des substances actives tant hydrophiles que lipophiles sont envisageables.

Le terme "qui n'est pas sous forme amorphe" doit être compris selon l'acceptation classique du terme amorphe. Différentes sources donnent la définition de ce terme "amorphe", signifiant non-cristallin, dépourvu de structure en réseau caractéristique de la cristallinité. Les références auxquelles renvoie la présent demande et donnant une définition du terme amorphe (ou de son contraire) sont de façon non-limitative: Hawley's, Condensed Chemical Dictionary, 12th Edition, p. 71; Handbook of Chemistry and Physics, 65th Edition, F-67; The Theory and Practice of Industrial Pharmacy, 1970, pp. 253-255; Remington's Pharmaceutical Sciences, 14th Edition, p. 182; General Chemistry, 1992, pp. 314-315; Encyclopedia of Pharmaceutical Technology, vol I, pp. 12-13.

Le terme "à l'exclusion de composés basiques" doit être compris comme excluant la présence d'un composé ou d'un groupe de composés qui confèrent un caractère basique à la composition, à savoir un pH supérieur à 7, lorsque la composition est diluée dans de l'eau à raison de 10g par litre d'eau. En particulier, ce terme entend exclure la présence d'un ou plusieurs composant(s) basique(s) tel(s) que décrit(s) colonne 1, lignes 38 à 62 du brevet US-P-4 404 183, si aucun composé acide ne vient contrebalancer l'effet dudit composé basique, ou si ce composé basique est présent en une quantité relativement importante.

Selon un mode de réalisation, la composition selon l'invention comprend:
(a) de 5 à 45 % de principe actif;
(b) de 25 à 70 % de polyéthylèneoxyde;
(c) la balance étant constituée d'additifs classiques, à l'exclusion de composants basiques.

Selon un mode de réalisation, dans la composition selon l'invention, le principe actif est un principe actif hydrophile ou lipophile, avantageusement hydrophile.

Selon un autre mode de réalisation, dans la composition selon l'invention, le principe actif est choisi dans le groupe consistant en acyclovir, nifédipine, nicardipine, captopril, vérapamil, diltiazem, oxybutynine, valacyclovir, glipizide, felodipine, isosorbide, carbidopa, levodopa, pentoxiphylline, et leurs sels pharmaceutiquement acceptables.

Selon une variante, dans la composition selon l'invention le polyéthylèneoxyde présente un poids moléculaire qui varie de 50 000 à 8 000 000, de préférence de 100 000 à 3 000 000. Le poids moléculaire souhaité pour le PEO peut être obtenu par mélange de PEO de divers poids moléculaires disponibles dans le commerce.

Selon une autre variante, dans la composition selon l'invention, la balance constituée d'additifs classiques comprend de la cellulose microcristalline, du lactose, de l'acide ascorbique, des pigments, des plastifiants, des lubrifiants et autres. Bien entendu, d'autres additifs classiques connus de l'homme de l'art peuvent être utilisés.

Selon un mode de réalisation de l'invention, dans la composition, la balance constituée d'additifs classiques comprend du stéarate de magnésium et/ou du béhénate de glycérol et/ou du sodium stéaryl fumarate, utilisé à titre de lubrifiant permettant une meilleure compression de la composition en des comprimés par exemple.

Selon une variante de l'invention, la composition est de plus enrobée. Cet enrobage ou revêtement est utilisé à des fins d'amélioration d'apparence pour faciliter l'acceptation par le patient, ou de stabilité dimensionnelle du comprimé. L'enrobage peut aussi être un enrobage entérique classique. L'enrobage est obtenu par tout procédé classique, avec des ingrédients classiques. Le revêtement peut par exemple être obtenu à l'aide d'un film à dissolution rapide. Il convient de noter que l'enrobage selon la présente invention est fondamentalement distinct du revêtement selon la demande de brevet EP-A-0 277 092, puisqu'on ne retrouve pas dans l'invention cette dichotomie (noyau expansif à l'eau)/ (revêtement poreux à l'eau), et que de plus l'enrobage selon l'invention se dissout et/ou se désintègre, alors que l'enrobage selon la demande de brevet EP-A-0 277 092 ne se dissout pas.

Les présentes compositions solides sont appropriées pour l'administration de médicaments. Ainsi, l'invention a aussi pour objet les compositions pharmaceutiques en dérivant, ainsi que les compositions pour leur utilisation à titre de médicament.

La présente composition est susceptible d'être obtenue par le procédé comprenant les étapes de :
(i) mélange à l'état sec pendant une durée suffisante du principe actif, du PEO et éventuellement d'un ou plusieurs additifs;
(ii) éventuellement ajout du solvant lorsqu'il est utilisé puis mélange pendant une durée suffisante;
(iii) granulation par passage au travers d'un tamis approprié;
(iv) séchage des granulés ainsi formés pendant une durée suffisante;
(v) éventuellement ajout d'un ou plusieurs additifs, mélange à l'état sec pendant un temps suffisant et passage au travers d'un tamis approprié;
(vi) éventuellement ajout d'un ou plusieurs additifs et mélange à l'état sec pendant une durée suffisante;
(vii) compression du mélange obtenu à l'étape précédente en le comprimé recherché; et
(viii) éventuellement enrobage dudit comprimé.

Le solvant utilisé, lorsqu'il est utilisé, est de préférence un alcool. Ce solvant est éliminé par un séchage ayant lieu à un moment ou un autre du procédé, et ne se retrouve sensiblement pas dans la composition finale.

La sélection des durées de mélange, des appareils, des tamis et autres conditions opératoires entre dans le champ de compétences de l'homme du métier.

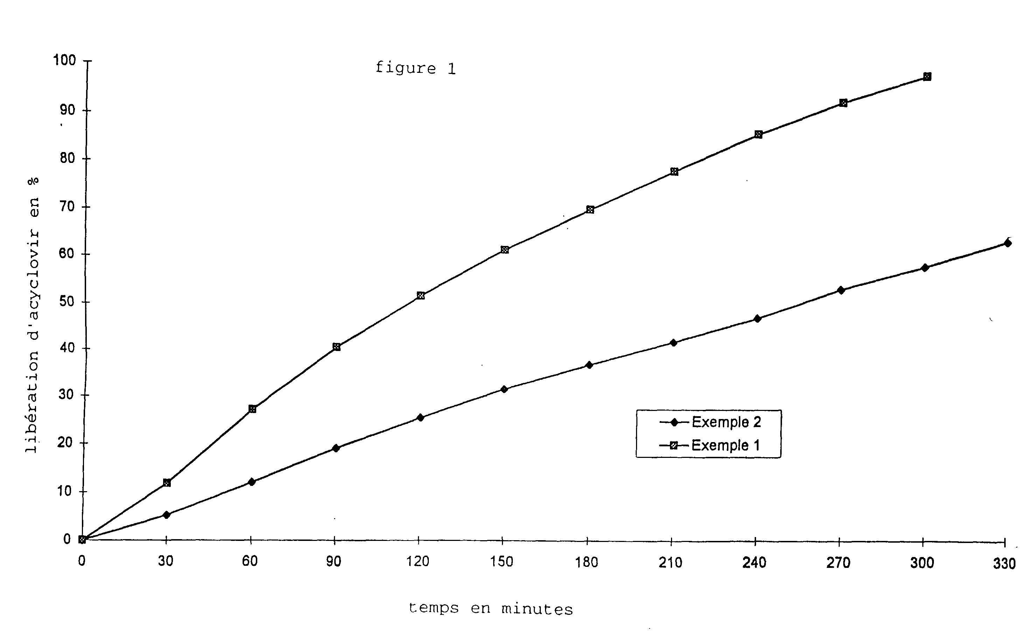

L'invention est maintenant décrite plus en détails par la description qui suit, en référence à la figure 1 unique qui donne la dissolution in vitro en % de libération du principe actif en fonction du temps pour les compositions solides des exemples 1 et 2.

Sans vouloir être liée par aucune théorie, la demanderesse pense que le PEO, dans la formulation, forme au contact de l'eau, un hydrogel. Cet hydrogel se dissout plus ou moins rapidement en fonction du poids moléculaire du PEO utilisé. Le choix du poids moléculaire de PEO, en combinaison avec le choix des concentrations pondérales du principe actif, du PEO, et des additifs, permet de contrôler la libération du principe actif.

Par ailleurs, la présente composition présence des résultats particulièrement surprenants. Dans une matrice hydrophile, lorsque la concentration en principe actif hydrophile augmente, on s'attend à ce que la vitesse de libération du principe actif augmente. La présente composition présente l'effet inverse, dans le cas par exemple de l'Acyclovir comme principe actif. Ceci apparaît clairement de la figure 1, qui donne la dissolution in vitro des compositions des exemples 1 et 2. La composition de l'exemple 1 contient une concentration inférieure en principe actif (200 mg de médicament dans un comprimé de 905 mg) et montre une libération in vitro plus rapide que la composition de l'exemple 2 qui contient une concentration supérieure en principe actif (400 mg de médicament dans un comprimé de 905 mg). Ce résultat est particulièrement surprenant.

Les exemples suivants sont donnés à titre illustratif de l'invention et ne doivent pas être considérés comme limitatifs de la portée de celle-ci. Dans les exemples, on indique entre parenthèses la quantité de solvant utilisée, étant entendu que ce solvant ne se retrouve sensiblement pas dans la composition finale.

### Exemple 1:

On prépare la composition suivante:

| | |
|---|---|
| Acyclovir | 200.0 mg |
| PEO (PM = 100 000) | 700.0 mg |
| Stéarate de magnésium | 5.0 mg |
| Alcool industriel | (260.0 mg) |

On pèse et on ajoute l'Acyclovir et le PEO dans un malaxeur. On mélange à l'état sec pendant 5 minutes. On ajoute l'alcool au mélange et on malaxe pendant une durée de 5 mn. On granule par passage au travers d'un tamis d'une dimension de 1,6 mm. On sèche les granulats. On les fait passer au travers d'un tamis d'une dimension de 0,8 mm. On pèse et on ajoute le stéarate de magnésium, et on mélange à l'état sec pendant 2 minutes. Les comprimés sont obtenus par compression dans une machine Frogerais MR 15.

### Exemple 2:

On prépare la composition suivante:

| | |
|---|---|
| Acyclovir | 400.0 mg |
| PEO (PM = 100 000) | 500.0 mg |
| Stéarate de magnésium | 5.0 mg |
| Alcool industriel | (260.0 mg) |

On pèse et on ajoute l'Acyclovir et le PEO dans un malaxeur. On mélange à l'état sec pendant 5 minutes. On ajoute l'alcool au mélange et on malaxe pendant une durée de 5 mn. On granule par passage au travers d'un tamis d'une dimension de 1,6 mm. On sèche les granulats. On les fait passer au travers d'un tamis d'une dimension de 0,8 mm. On pèse et on ajoute le stéarate de magnésium, et on mélange à l'état sec pendant 2 minutes. Les comprimés sont obtenus par compression dans une machine rotative de type Frogerais MR 15.

### Exemple 3:

On prépare la composition suivante:

| | |
|---|---|
| Nifédipine | 60.0 mg |
| Cellulose microcristalline | 100.0 mg |
| PEO (PM = 3 000 000) | 336.0 mg |
| Dioxyde de silicium colloïdal | 2.5 mg |
| Stéarate de magnésium | 2.5 mg |
| Alcool industriel | (150.0 mg) |

On pèse et on ajoute la nifédipine, la cellulose microcristalline (disponible chez FMC sous le nom d'Avicel PH 101) et le PEO dans un malaxeur. On mélange à l'état sec pendant 5 minutes. On ajoute l'alcool au mélange et on malaxe pendant une durée de 5 mn. On granule par passage au travers d'un tamis d'une dimension de 1,8 mm. On sèche les granulats. On pèse et on ajoute le dioxyde de silicium colloïdal (disponible chez Degussa sous le nom d'Aérosil 200), on mélange à l'état sec pendant 5 mn, et on passe au travers d'un tamis d'une dimension de 0,6 mm. On pèse et on ajoute le stéarate de magnésium, et on mélange à l'état sec pendant 2 minutes. Les comprimés sont obtenus par compression dans une machine rotative de type Frogerais MR 15.

### Exemple 4:

On prépare la composition suivante:

| Noyau. | |
|---|---|
| Nifédipine | 60.0 mg |
| Cellulose microcristalline | 100.0 mg |
| PEO (PM = 3 000 000) | 336.0 mg |
| Dioxyde de silicium colloïdal | 2.5 mg |
| Stéarate de magnésium | 2.5 mg |
| Alcool industriel | (150.0 mg) |

| Revêtement. | |
|---|---|
| Oxyde de fer | 2.0 mg |
| Dioxyde de titane | 1.0 mg |
| Méthylcellulose | 12.0 mg |
| Alcool industriel | (150.0 mg) |

On pèse et on ajoute la nifédipine, la cellulose microcristalline (disponible chez FMC sous le nom d'Avicel PH 101) et le PEO dans un malaxeur. On mélange à l'état sec pendant 5 minutes. On ajoute l'alcool au mélange et on malaxe pendant une durée de 5 mn. On granule par passage au travers d'un tamis d'une dimension de 1,6 mm. On sèche les granulats. On pèse et on ajoute le dioxyde de silicium colloïdal (disponible chez Degussa sous le nom d'Aérosil 200), on mélange à l'état sec pendant 10 mm, et on passe au travers d'un tamis d'une dimension de 0,8 mm. On pèse et on ajoute le stéarate de magnésium, et on mélange à l'état sec pendant 2 minutes. Les comprimés sont obtenus par compression dans une machine rotative de type Frogerais MR 15.

On dissout ensuite la méthylcellulose (disponible chez Colorcon sous le nom de Methocel) dans l'alcool. On ajoute l'oxyde de fer et le dioxyde de titane dans la solution et on homogénéise dans un appareil Ultra Turrax pendant 10 mn. Les comprimés sont revêtus par pulvérisation de cette suspension dans un appareil d'enrobage à "perforated pan" de type "Glatt coater".

### Exemple 5:

On prépare la composition suivante:

| Noyau. | |
|---|---|
| Nicardipine HCl | 60.0 mg |
| Cellulose microcristalline | 77.0 mg |
| PEO (PM = 2 000 000) | 270.0 mg |
| Stéarate de magnésium | 3.0 mg |
| Alcool industriel | (150.0 mg) |

| Revêtement. | |
|---|---|
| Oxyde de fer | 2.0 mg |
| Dioxyde de titane | 1.0 mg |
| Méthylcellulose | 12.0 mg |
| Alcool industriel | (150.0 mg) |

On pèse et on ajoute la nicardipine. HCl, la cellulose microcristalline (Avicel PH 101) et le PEO dans un malaxeur. On mélange à l'état sec pendant 5 minutes. On ajoute l'alcool au mélange et on malaxe pendant une durée de 5 mn. On granule par passage au travers d'un tamis d'une dimension de 1,6 mm. On sèche les granulats et on passe au travers d'un tamis d'une dimension de 0,8 mm. On pèse et on ajoute le stéarate de magnésium, et on mélange à l'état sec pendant 2 minutes. Les comprimés sont obtenus par compression dans une machine rotative de type Frogerais MR 15.

On dissout ensuite la méthylcellulose (Methocel) dans l'alcool. On ajoute l'oxyde de fer et le dioxyde de titane dans la solution et on homogénéise dans un appareil Ultra Turrax pendant 10 mn. Les comprimés sont revêtus par pulvérisation de cette suspension dans un appareil d'enrobage de type "Glatt coater".

### Exemple 6:

On prépare la Composition suivante:

| Noyau. | |
|---|---|
| Captopril | 50.0 mg |
| Cellulose microcristalline | 100.0 mg |
| PEO (PM = 2 500 000) | 300.0 mg |
| Acide ascorbique (poudre) | 100.0 mg |
| Stéarate de magnésium | 3.0 mg |

| Revêtement. | |
|---|---|
| Dioxyde de titane | 1.0 mg |
| Méthylcellulose | 10.0 mg |
| Alcool industriel | (150.0 mg) |

On pese et on ajoute le captopril, la cellulose microcristalline (Avicel 200), l'acide ascorbique et le PEO dans un malaxeur. On mélange à l'état sec pendant 5 minutes. On passe au travers d'un tamis d'une dimension de 1,6 mm. On pèse et on ajoute le stéarate de magnésium, et on mélange à l'état sec pendant 2 minutes. Les comprimés sont obtenus par compression dans une machine rotative de type Frogerais MR 15.

On dissout ensuite la méthylcellulose (Methocel) dans l'alcool. On ajoute le dioxyde de titane dans la solution et on homogénéise dans un appareil Ultra Turrax pendant 10 mn. Les comprimés sont revêtus par pulvérisation de cette suspension dans un appareil d'enrobage de type "Glatt coater".

### Exemple 7:

On prépare la composition suivante:

| | |
|---|---|
| Vérapamil.HCl | 240.0 mg |
| Lactose | 100.0 mg |
| PEO (PM = 1 000 000) | 200.0 mg |
| Stéarate de magnésium | 5.0 mg |
| Alcool industriel | (200.0 mg) |

On pèse et on ajoute le vérapamil.HCl, le lactose (disponible chez HMS sous la référence 80 mesh) et le PEO dans un malaxeur. On mélange à l'état sec pendant 5 minutes. On ajoute l'alcool au mélange et on malaxe pendant une durée de 5 mn. On granule par passage au travers d'un tamis d'une dimension de 1,6 mm. On sèche les granulats et on passe au travers d'un tamis d'une dimension de 0,8 mm. On pèse et on ajoute le stéarate de magnésium, et on mélange à l'état sec pendant 2 minutes. Les comprimés sont obtenus par compression dans une machine rotative de type Frogerais MR 15.

### Exemple 8:

On prépare la composition suivante:

| | |
|---|---|
| Diltiazem HCl | 180.0 mg |
| Lactose | 100.0 mg |
| PEO (PM = 1 500 000) | 160.0 mg |
| Stéarate de magnésium | 3.0 mg |
| Alcool industriel | (150.0 mg) |

On pèse et on ajoute le diltiazem.HCl, le lactose (HMS, 80 mesh) et le PEO dans un malaxeur. On mélange à l'état sec pendant 5 minutes. On ajoute l'alcool au mélange et on malaxe pendant une durée de 5 mn. On granule par passage au travers d'un tamis d'une dimension de 1,6 mm. On sèche les granulats et on passe au travers d'un tamis d'une dimension de 0,8 mm. On pèse et on ajoute le stéarate de magnésium, et on mélange à l'état sec pendant 2 minutes. Les comprimés sont obtenus par compression dans une machine rotative de type Frogerais MR 15.

### Exemple 9:

On prépare la composition suivante:

| | |
|---|---|
| Oxybutynine.HCl | 15.0 mg |
| Cellulose microcristalline | 75.0 mg |
| PEO (PM = 1 000 000) | 120.0 mg |
| Dioxyde de silicium colloïdal | 1.5 mg |
| Stéarate de magnésium | 1.5 mg |
| Alcool industriel | (110.0 mg) |

On pèse et on ajoute l'oxybutynine.HCl, la cellulose microcristalline (Avicel PH 101) et le PEO dans un malaxeur. On mélange à l'état sec pendant 5 minutes. On ajoute l'alcool au mélange et on malaxe pendant une durée de 5 mn. On granule par passage au travers d'un tamis d'une dimension de 1,6 mm. On sèche les granulats. On pèse et on ajoute le dioxyde de silicium colloïdal (Aérosil 200), on mélange à l'état sec pendant 10 mn, et on passe au travers d'un tamis d'une dimension de 0,8 mm. On pèse et on ajoute le stéarate de magnésium, et on mélange à l'état sec pendant 2 minutes. Les comprimés sont obtenus par compression dans une machine rotative de type Frogerais MR 15.

### Exemple 10:

On prépare la composition suivante:

| Noyau. | |
|---|---|
| Nifédipine | 60.0 mg |
| Cellulose microcristalline | 100.0 mg |
| PEO (PM = 3 000 000) | 336.0 mg |
| Dioxyde de silicium colloïdal | 2.5 mg |
| Stéarate de magnésium | 2.5 mg |
| Alcool industriel | (150.0 mg) |

| Revêtement 1. | |
|---|---|
| Copolymère Ammonio Methacrylate Type A | 160.0 mg |
| Hydroxypropylméthylcellulose | 20.0 mg |
| eau | 30.0 mg |
| Alcool industriel | (150.0 mg) |

| Revêtement 2. | |
|---|---|
| Oxyde de fer | 2.0 mg |
| Dioxyde de titane | 1.0 mg |
| Méthylcellulose | 12.0 mg |
| Alcool industriel | (150.0 mg) |

Le procédé de préparation du noyau est identique à celui de l'exemple 4.

Le procédé de préparation du revêtement 1 est le suivant, On pèse et on dissout l'hydroxypropylméthylcellulose dans le mélange eau/alcool, On pèse et on ajoute le copolymère amminio méthacrylate (USP XXIII, 12,5% de solide, disponible sous le nom de Eudragit RL chez Rohm Pharma, Allemagne) et on mélange. Le revêtement est effectué dans un dispositif de type "Glatt coater".

Le procédé de préparation du revêtement 2 et le revêtement du comprimé obtenu à l'étape précédente sont identiques à ceux de l'exemple 4.

### Exemple 11:

On prépare la composition suivante:

| | |
|---|---|
| Valacyclovir | 200.0 mg |
| PEO (PM = 300 000) | 700.0 mg |
| Stéarate de magnésium | 5.0 mg |
| Alcool industriel | (260.0 mg) |

On pèse et on ajoute le Valacyclovir et le PEO dans un malaxeur. On mélange à l'état sec pendant 5 minutes. On ajoute l'alcool au mélange et on malaxe pendant une durée de 5 mn. On granule par passage au travers d'un tamis d'une dimension de 1,6 mm. On sèche les granulats. On les fait passer au travers d'un tamis d'une dimension de 0,8 mm. On pèse et on ajoute le stéarate de magnésium, et on mélange à l'état sec pendant 2 minutes. Les comprimés sont obtenus par compression dans une machine de type Frogerais MR 15.

Suivant le même procédé que dans les exemples précédents, on prépare les compositions suivantes, dans lesquelles le principe actif est sous forme de poudre cristalline.

### Exemple 12:

On prépare la composition suivante:

| Noyau | |
|---|---|
| Glipizide | 10.0 mg |
| PEO | 220.0 mg |
| Cellulose microcristalline | 55.0 mg |
| Hydroxypropylméthylcellulose | 20.0 mg |
| Lactose | 50.0 mg |
| Sodium stéaryl fumarate | 1.7 mg |

| Revêtement | |
|---|---|
| Copolymère acide méthacrylique | 10.0 mg |
| Polyéthylène glycol | 2.0 mg |
| Talc | 2.5 mg |
| Dioxyde de silicium | 4.5 mg |

### Exemple 13:

On prépare la composition suivante:

| Noyau | |
|---|---|
| Glipizide | 10.0 mg |
| PEO | 220.0 mg |
| Cellulose microcristalline | 55.0 mg |
| Hydroxypropylméthylcellulose | 20.0 mg |
| Lactose | 50.0 mg |
| Sodium stéaryl fumarate | 1.7 mg |

| Revêtement | |
|---|---|
| Copolymère ammonio méthacrylate | 10.0 mg |
| Triéthyl citrate | 3.0 mg |
| Polyéthylène glycol | 1.0 mg |
| Hydroxypropylméthylcellulose | 7.0 mg |

### Exemple 14:

On prépare la composition suivante.

| Noyau | |
|---|---|
| Glipizide | 10.0 mg |
| PEO | 220.0 mg |
| Cellulose microcristalline | 55.0 mg |
| Hydroxypropylméthylcellulose | 20.0 mg |
| Lactose | 50.0 mg |
| Sodium stéaryl fumarate | 1.7 mg |

| Revêtement | |
|---|---|
| Copolymère ammonio méthacrylate | 10.0 mg |
| Lactose | 10.0 mg |
| Dioxyde de silicium | 4.0 mg |

### Exemple 15:

On prépare la composition suivante.

| Noyau | |
|---|---|
| Glipizide | 10.0 mg |
| PEO | 220.0 mg |
| Cellulose microcristalline | 55.0 mg |
| Hydroxypropylméthylcellulose | 20.0 mg |
| Lactose | 50.0 mg |
| Sodium stéaryl fumarate | 1.7 mg |

| Revêtement | |
|---|---|
| Dispersion à 30% de polyacrylate | 15.0 mg |
| Dioxyde de silicium | 6.0 mg |
| Talc | 2.0 mg |
| Hydroxypropylméthylcellulose | 6.0 mg |

### Exemple 16:

On prépare la composition suivante.

| Noyau | |
|---|---|
| Felodipine | 10.0 mg |
| PEO | 220.0 mg |
| Cellulose microcristalline | 55,0 mg |
| Hydroxypropylméthylcellulose | 20.0 mg |
| Sodium stéaryl fumarate | 1.5 mg |

| Revêtement | |
|---|---|
| Copolymère acide méthacrylique | 10.0 mg |
| Polyéthylène glycol | 2.0 mg |
| Talc | 2.5 mg |
| Dioxyde de silicium | 4.5 mg |

### Exemple 17:

On prépare la composition suivante.

| Noyau | |
|---|---|
| Felodipine | 10.0 mg |
| PEO | 220.0 mg |
| Cellulose microcristalline | 55.0 mg |
| Hydroxypropylméthylcellulose | 20.0 mg |
| Sodium stéaryl fumarate | 1.5 mg |

| Revêtement | |
|---|---|
| Copolymère ammonio méthacrylate | 10.0 mg |
| Triéthyl citrate | 3.0 mg |
| Polyéthylène glycol | 1.0 mg |
| Hydroxypropylméthylcellulose | 7.0 mg |

### Exemple 18:

On prépare la composition suivante.

| Noyau | |
|---|---|
| Felodipine | 10.0 mg |
| PEO | 220.0 mg |
| Cellulose microcristalline | 55.0 mg |
| Hydroxypropylméthylcellulose | 20.0 mg |
| Sodium stéaryl fumarate | 1.5 mg |

| Revêtement | |
|---|---|
| Copolymère ammonio méthacrylate | 10.0 mg |
| Lactose | 10.0 mg |
| Dioxyde de silicium | 4.0 mg |

### Exemple 19:

On prépare la composition suivante.

| Noyau | |
|---|---|
| Felodipine | 10.0 mg |
| PEO | 220.0 mg |
| Cellulose microcristalline | 55.0 mg |
| Hydroxypropylméthylcellulose | 20.0 mg |
| Sodium stéaryl fumarate | 1.5 mg |

| Revêtement | |
|---|---|
| Dispersion à 30% de polyacrylate | 15.0 mg |
| Dioxyde de silicium | 6.0 mg |
| Talc | 2.0 mg |
| Hydroxypropylméthylcellulose | 6.0 mg |

### Exemple 20:

On prépare la composition suivante.

| Noyau | |
|---|---|
| Isosorbide mononitrate | 60.0 mg |
| PEO | 100.0 mg |
| Cellulose microcristalline | 25.0 mg |
| Hydroxypropylcellulose faiblement substituée | 5.0 mg |
| Glycérol béhénate | 1.9 mg |

| Revêtement | |
|---|---|
| Copolymère acide méthacrylique | 10.0 mg |
| Polyéthylène glycol | 2.0 mg |
| Talc | 2.5 mg |
| Dioxyde de silicium | 4.5 mg |

### Exemple 21:

On prépare la composition suivante.

| Noyau | |
|---|---|
| Isosorbide mononitrate | 60.0 mg |
| PEO | 100.0 mg |
| Cellulose microcristalline | 25.0 mg |
| Hydroxypropylcellulose faiblement substituée | 5.0 mg |
| Glycérol béhénate | 1.9 mg |

| Revêtement | |
|---|---|
| Copolymère ammonio méthacrylate | 5.0 mg |
| Triéthyl citrate | 1.5 mg |
| Polyéthylène glycol | 0.5 mg |
| Hydroxypropylméthylcellulose | 3.5 mg |

### Exemple 22:

On prépare la composition suivante.

| Noyau | |
|---|---|
| Isosorbide mononitrate | 60.0 mg |
| PEO | 100.0 mg |
| Cellulose microcristalline | 25.0 mg |
| Hydroxypropylcellulose faiblement substituée | 5.0 mg |
| Glycérol béhénate | 1.9 mg |

| Revêtement | |
|---|---|
| Copolymère ammonio méthacrylate | 5.0 mg |
| Lactose | 5.0 mg |
| Dioxyde de silicium | 2.0 mg |

### Exemple 23:

On prépare la composition suivante.

| Noyau | |
|---|---|
| Isosorbide mononitrate | 60.0 mg |
| PEO | 100.0 mg |
| Cellulose microcristalline | 25.0 mg |
| Hydroxypropylcellulose faiblement substituée | 5.0 mg |
| Glycérol béhénate | 1.9 mg |

| Revêtement | |
|---|---|
| Dispersion à 30% de polyacrylate | 7.5 mg |
| Dioxyde de silicium | 3.0 mg |
| Talc | 3.0 mg |
| Hydroxypropylméthylcellulose | 3.0 mg |

### Exemple 24:

On prépare la composition suivante.

| Noyau | |
|---|---|
| Carbidopa | 25.0 mg |
| Levodopa | 100.0 mg |
| Cellulose microcristalline | 20.0 mg |
| Povidone | 4.0 mg |
| Hydroxypropylcellulose faiblement substituée | 8.0 mg |
| PEO | 20.0 mg |
| Sodium stéaryl fumarate | 1.7 mg |

| Revêtement | |
|---|---|
| Copolymère acide méthacrylique | 5.0 mg |
| Polyéthylène glycol | 1.0 mg |
| Talc | 1.25mg |
| Dioxyde de silicium | 2.25mg |

### Exemple 25:

On prépare la composition suivante.

| Noyau | |
|---|---|
| Carbidopa | 25.0 mg |
| Levodopa | 100.0 mg |
| Cellulose microcristalline | 20.0 mg |
| Povidone | 4.0 mg |
| Hydroxypropylcellulose faiblement substituée | 8.0 mg |
| PEO | 20.0 mg |
| Sodium stéaryl fumarate | 1.7 mg |

| Revêtement | |
|---|---|
| Copolymère ammonio méthacrylate | 5.0 mg |
| Triéthyl citrate | 1.5 mg |
| Polyéthylène glycol | 0.5 mg |
| Hydroxypropylméthylcellulose | 3.5 mg |

### Exemple 26:

On prépare la composition suivante.

| Noyau | |
|---|---|
| Carbidopa | 25.0 mg |
| Levodopa | 100.0 mg |
| Cellulose microcristalline | 20.0 mg |
| Povidone | 4.0 mg |
| Hydroxypropylcellulose faiblement substituée | 8.0 mg |
| PEO | 20.0 mg |
| Sodium stéaryl fumarate | 1.7 mg |

| Revêtement | |
|---|---|
| Copolymère ammonio méthacrylate | 5.0 mg |
| Lactose | 5.0 mg |
| Dioxyde de silicium | 2.0 mg |

### Exemple 27:

On prépare la composition suivante.

| Noyau | |
|---|---|
| Carbidopa | 25.0 mg |
| Levodopa | 100.0 mg |
| Cellulose microcristalline | 20.0 mg |
| Povidone | 4.0 mg |
| Hydroxypropylcellulose faiblement substituée | 8.0 mg |
| PEO | 20.0 mg |
| Sodium stéaryl fumarate | 1.7 mg |

| Revêtement | |
|---|---|
| Dispersion à 30% de polyacrylate | 7.5 mg |
| Dioxyde de silicium | 3.0 mg |
| Talc | 1.0 mg |
| Hydroxypropylméthylcellulose | 3.0 mg |

### Exemple 28:

On prépare la composition suivante.

| Noyau | |
|---|---|
| Pentoxiphylline | 400.0 mg |
| PEO | 150.0 mg |
| Povidone | 30.0 mg |
| Glycérol béhénate | 6.0 mg |

| Revêtement | |
|---|---|
| Copolymère acide méthacrylique | 20.0 mg |
| Polyéthylène glycol | 4.0 mg |
| Talc | 5.0 mg |
| Dioxyde de silicium | 9.0 mg |

### Exemple 29:

On prépare la composition suivante.

| Noyau | |
|---|---|
| Pentoxiphylline | 400.0 mg |
| PEO | 150.0 mg |
| Povidone | 30.0 mg |
| Glycérol béhénate | 6.0 mg |

| Revêtement | |
|---|---|
| Copolymère ammonio méthacrylate | 20.0 mg |
| Triéthyl citrate | 6.0 mg |
| Polyéthylène glycol | 2.0 mg |
| Hydroxypropylméthylcellulose | 14.0 mg |

### Exemple 30:

On prépare la composition suivante.

| Noyau | |
|---|---|
| Pentoxiphylline | 400.0 mg |
| PEO | 150.0 mg |
| Povidone | 30.0 mg |
| Glycérol béhénate | 6.0 mg |

| Revêtement | |
|---|---|
| Copolymère ammonio méthacrylate | 20.0 mg |
| Lactose | 20.0 mg |
| Dioxyde de silicium | 8.0 mg |

### Exemple 31:

On prépare la composition suivante.

| Noyau | |
|---|---|
| Pentoxiphylline | 400.0 mg |
| PEO | 150.0 mg |
| Povidone | 30.0 mg |
| Glycérol béhénate | 6.0 mg |

| Revêtement | |
|---|---|
| Dispersion à 30% de polyacrylate | 30.0 mg |
| Dioxyde de silicium | 12.0 mg |
| Talc | 4.0 mg |
| Hydroxypropylméthylcellulose | 12.0 mg |

### Exemple 32:

On prépare la composition suivante.

| Noyau | |
|---|---|
| Nicardipine | 30.0 mg |
| PEO | 150.0 mg |
| Cellulose microcristalline | 30.0 mg |
| Povidone | 5.0 mg |
| Stéarate de magnésium | 2.0 mg |

| Revêtement | |
|---|---|
| Copolymère acide méthacrylique | 6.0 mg |
| Polyéthylène glycol | 1.2 mg |
| Talc | 1.5 mg |
| Dioxyde de silicium | 2.7 mg |

### Exemple 33:

On prépare la composition suivante.

| Noyau | |
|---|---|
| Nicardipine | 30.0 mg |
| PEO | 150.0 mg |
| Cellulose microcristalline | 30.0 mg |
| Povidone | 5.0 mg |
| Stéarate de magnésium | 2.0 mg |

| Revêtement | |
|---|---|
| Copolymère ammonio méthacrylate | 6.0 mg |
| Triéthyl citrate | 1.8 mg |
| Polyéthylène glycol | 0.6 mg |
| Hydroxypropylméthylcellulose | 4.2 mg |

### Exemple 34:

On prépare la composition suivante.

| Noyau | |
|---|---|
| Nicardipine | 30.0 mg |
| PEO | 150.0 mg |
| Cellulose microcristalline | 30.0 mg |
| Povidone | 5.0 mg |
| Stéarate de magnésium | 2.0 mg |

| Revêtement | |
|---|---|
| Copolymère ammonio méthacrylate | 6.0 mg |
| Lactose | 6.0 mg |
| Dioxyde de silicium | 2.4 mg |

### Exemple 35:

On prépare la composition suivante.

| Noyau | |
|---|---|
| Nicardipine | 30.0 mg |
| PEO | 150.0 mg |
| Cellulose microcristalline | 30.0 mg |
| Povidone | 5.0 mg |
| Stéarate de magnésium | 2.0 mg |

| Revêtement | |
|---|---|
| Dispersion à 30% de polyacrylate | 9.0 mg |
| Dioxyde de silicium | 3.6 mg |
| Talc | 1.2 mg |
| Hydroxypropylméthylcellulose | 3.6 mg |

## Revendications

1. Composition solide comprenant, en poids sur la base du poids total de la composition:
(a) de 1 à 70 % d'un principe actif qui n'est pas sous forme amorphe;
(b) de 10 à 95 % de polyéthylèneoxyde;
(c) la balance étant constituée d'additifs classiques, à l'exclusion de composants basiques,
ladite composition étant susceptible d'être obtenue par le procédé comprenant les étapes de :
(i) mélange à l'état sec pendant une durée suffisante du principe actif, du polyéthylèneoxyde et éventuellement d'un ou plusieurs additifs;
(ii) éventuellement ajout du solvant lorsqu'il est utilisé puis mélange pendant une durée suffisante;
(iii) granulation par passage au travers d'un tamis approprié;
(iv) séchage des granulés ainsi formés pendant une durée suffisante;
(v) éventuellement ajout d'un ou plusieurs additifs, mélange à l'état sec pendant un temps suffisant et passage au travers d'un tamis approprié;
(vi) éventuellement ajout d'un ou plusieurs additifs et mélange à l'état sec pendant une durée suffisante;
(vii) compression du mélange obtenu à l'étape précédente en le comprimé recherché; et
(viii) éventuellement enrobage dudit comprimé.

2. Composition selon la revendication 1, comprenant:
(a) de 5 à 45 % de principe actif;
(b) de 25 à 70 % de polyéthylèneoxyde;
(c) la balance étant constituée d'additifs classiques, à l'exclusion de composants basiques.

3. Composition selon la revendication 1 ou 2, dans laquelle le principe actif est un principe actif hydrophile ou lipophile.

4. Composition selon la revendication 3, dans laquelle le principe actif est un principe actif hydrophile.

5. Composition selon l'une quelconque des revendications 1 à 4, dans laquelle le principe actif est l'acyclovir ou un sel pharmaceutiquement acceptable.

6. Composition selon l'une quelconque des revendications 1 à 4, dans laquelle le principe actif est la nifédipine ou un sel pharmaceutiquement acceptable.

7. Composition selon l'une quelconque des revendications 1 à 4, dans laquelle le principe actif est la nicardipine ou un sel pharmaceutiquement acceptable.

8. Composition selon l'une quelconque des revendications 1 à 4, dans laquelle le principe actif est le captopril ou un sel pharmaceutiquement acceptable.

9. Composition selon l'une quelconque des revendications 1 à 4, dans laquelle le principe actif est le vérapamil ou un sel pharmaceutiquement acceptable.

10. Composition selon l'une quelconque des revendications 1 à 4, dans laquelle le principe actif est le diltiazem ou un sel pharmaceutiquement acceptable.

11. Composition selon l'une quelconque des revendications 1 à 4, dans laquelle le principe actif est l'oxybutynine ou un sel pharmaceutiquement acceptable.

12. Composition selon l'une quelconque des revendications 1 à 4, dans laquelle le principe actif est le valacyclovir ou un sel pharmaceutiquement acceptable.

13. Composition selon l'une quelconque des revendications 1 à 4, dans laquelle le principe actif est le glipizide ou un sel pharmaceutiquement acceptable.

14. Composition selon l'une quelconque des revendications 1 à 4, dans laquelle le principe actif est la felodipine ou un sel pharmaceutiquement acceptable.

15. Composition selon l'une quelconque des revendications 1 à 4, dans laquelle le principe actif est l'isosorbide ou un sel pharmaceutiquement acceptable.

16. Composition selon l'une quelconque des revendications 1 à 4, dans laquelle le principe actif est le carbidopa ou un sel pharmaceutiquement acceptable.

17. Composition selon l'une quelconque des revendications 1 à 4, dans laquelle le principe actif est le levodopa ou un sel pharmaceutiquement acceptable.

18. Composition selon l'une quelconque des revendications 1 à 4, dans laquelle le principe actif est la pentoxiphylline ou un sel pharmaceutiquement acceptable.

19. Composition selon l'une quelconque des revendications 1 à 18, dans laquelle le polyéthylèneoxyde présente un poids moléculaire qui varie de 50 000 à 8 000 000, de préférence de 100 000 à 3 000 000.

20. Composition selon l'une quelconque des revendications 1 à 19, dans laquelle la balance constituée d'additifs classiques comprend de la cellulose microcristalline, du lactose, des pigments, des plastifiants, des lubrifiants, et autres.

21. Composition selon l'une quelconque des revendications 1 à 20, dans laquelle la balance constituée d'additifs classiques comprend du stéarate de magnésium et/ou du béhénate de glycérol et/ou du sodium stéaryl fumarate.

22. Composition selon l'une quelconque des revendications 1 à 21, qui est de plus enrobée.

23. Composition selon l'une quelconque des revendications 1 à 22, qui est une composition pharmaceutique.

24. Composition selon l'une quelconque des revendications 1 à 23, pour son utilisation à titre de médicament.

25. Procédé de préparation d'une composition selon l'une quelconque des revendications 1 à 24, comprenant les étapes de :
(i) mélange à l'état sec pendant une durée suffisante du principe actif, du polyéthylèneoxyde et éventuellement d'un ou plusieurs additifs;
(ii) éventuellement ajout du solvant lorsqu'il est utilisé puis mélange pendant une durée suffisante;
(iii) granulation par passage au travers d'un tamis approprié;
(iv) séchage des granulés ainsi formés pendant une durée suffisante;
(v) éventuellement ajout d'un ou plusieurs additifs, mélange à l'état sec pendant un temps suffisant et passage au travers d'un tamis approprié;
(vi) éventuellement ajout d'un ou plusieurs additifs et mélange à l'état sec pendant une durée suffisante;
(vii) compression du mélange obtenu à l'étape précédente en le comprimé recherché; et
(viii) éventuellement enrobage dudit comprimé.

26. Procédé selon la revendication 25, dans lequel le solvant utilisé, lorsqu'un tel solvant est utilisé, est un alcool.

## Claims

1. A solid pharmaceutical composition comprising, by weight based on the total weight of the composition:
(a) from 1 to 70% of active ingredient which is not in an amorphous form ;
(b) from 10 to 95% of polyethylene oxide ;
(c) the balance consisting of conventional additives, excluding basic components,
said composition being obtained by the process comprising the steps of :
(i) mixing in the dry state and for a sufficient time, an active ingredient, polyethylene oxide and optionally, one or several additives ;
(ii) optionally adding solvent when such is used, followed by mixing for a sufficient period of time ;
(iii) granulation by passage through a suitable sieve;
(iv) drying the granules thus formed for a sufficient period of time;
(v) optionally adding one of more additives, with mixing in the dry state for a sufficient time and passage through a suitable sieve ;
(vi) optionally adding one or several additives and mixing in the dry state for a sufficient period of time ;
(vii) compressing the mixture obtained from the preceding steps to obtain the desired compressed tablet ; and
(viii) optionally coating said compressed tablet.

2. The composition according to claim 1, comprising :
(a) from 5 to 45% of the active ingredient ;
(b) from 25 to 70% polyethylene oxide ;
(c) the balance consisting of conventional additives, excluding basic components.

3. The composition according to claim 1 or 2, in which the active ingredient is a hydrophilic or lipophilic active ingredient.

4. The composition according to claim 3, in which the active ingredient is a hydrophilic active ingredient.

5. The composition according to any one of claims 1 to 4, in which the active ingredient is acyclovir or the pharmaceutically acceptable salts thereof.

6. The composition according to any one of claims 1 to 4, in which the active ingredient is nifedipine or the pharmaceutically acceptable salts thereof.

7. The composition according to any one of claims 1 to 4, in which the active ingredient is nicardipine or the pharmaceutically acceptable salts thereof.

8. The composition according to any one of claims 1 to 4, in which the active ingredient is captopril or the pharmaceutically acceptable salts thereof.

9. The composition according to any one of claims 1 to 4, in which the active ingredient is verapamil or the pharmaceutically acceptable salts thereof.

10. The composition according to any one of claims 1 to 4, in which the active ingredient is diltiazem or the pharmaceutically acceptable salts thereof.

11. The composition according to any one of claims 1 to 4, in which the active ingredient is oxybutynine or the pharmaceutically acceptable salts thereof.

12. The composition according to any one of claims 1 to 4, in which the active ingredient is valacyclovir or the pharmaceutically acceptable salts thereof.

13. The composition according to any one of claims 1 to 4, in which the active ingredient is glipizide or the pharmaceutically acceptable salts thereof.

14. The composition according to any one of claims 1 to 4, in which the active ingredient is felodipine or the pharmaceutically acceptable salts thereof.

15. The composition according to any one of claims 1 to 4, in which the active ingredient is isosorbide or the pharmaceutically acceptable salts thereof.

16. The composition according to any one of claims 1 to 4, in which the active ingredient is carbidopa or the pharmaceutically acceptable salts thereof.

17. The composition according to any one of claims 1 to 4, in which the active ingredient is levodopa or the pharmaceutically acceptable salts thereof.

18. The composition according to any one of claims 1 to 4, in which the active ingredient is pentoxiphylline or the pharmaceutically acceptable salts thereof.

19. The composition according to any one of claims 1 to 5, in which the polyethylene oxide has a molecular weight which varies from 50,000 to 8,000,000, and preferably from 100,000 to 3,000,000.

20. The composition according to any one of claim 1 to 19, in which the balance consisting of conventional additives comprises microcrystalline cellulose, lactose, pigments, plastifying agents, lubricants, and others.

21. The composition according to any one of claims 1 to 20, in which the balance consisting of conventional additives comprises magnesium stearate and/or glycerol behenate and/or sodium stearyl fumarate.

22. The composition according to any one of claims 1 to 21, wherein said composition is additionally coated.

23. The composition according to any one of claims 1 to 22, wherein said compostion is a pharmaceutical composition.

24. The composition according to any one of claims 1 to 23, for its use as a medicament.

25. A method for preparing a composition according to any one of claims 1 to 24, comprising the steps consisting of :
(i) mixing in the dry state and for a sufficient time, the active ingredient, polyethylene oxide and optionally, one or several additives ;
(ii) optionally adding solvent when such is used, followed by mixing for a sufficient period of time ;
(iii) granulation by passage through a suitable sieve ;
(iv) drying the granules thus formed for a sufficient period of time ;
(v) optionally adding one of more additives, with mixing in the dry state for a sufficient time and passage through a suitable sieve ;
(vi) optionally adding one or several additives and mixing in the dry state for a sufficient period of time ;
(vii) compressing the mixture obtained from the preceding steps to obtain the desired compressed tablet ; and
(viii) optionally coating said compressed tablet.

26. The method according to claim 25, in which the solvent employed, when solvent is used, is an alcohol.

## Patentansprüche

1. Feststoffzusammensetzung, die in Gewichtsteilen bezogen auf das Gesamtgewicht der Zusammensetzung umfasst:
(a) 1 bis 70 % eines nicht amorphen Wirkstoffes;
(b) 10 bis 95 % Polyethylenoxid;
(c) der Rest aus herkömmlichen Zusatzstoffen besteht mit der Ausnahme von basischen Bestandteilen,
wobei die Zusammensetzung über ein Verfahren erhältlich ist, das die Schritte umfasst:
(i) Mischen im trockenen Zustand des Wirkstoffes, des Polyethylenoxids und gegebenenfalls eines oder mehrerer Zusatzstoffe für eine ausreichende Zeitdauer;
(ii) gegebenenfalls Hinzufügen von Lösungsmittel, falls ein solches verwendet wird, Mischen für eine ausreichende Zeitdauer;
(iii) Granulieren durch Passagieren durch ein geeignetes Sieb hindurch;
(iv) Trocknen der derartig gebildeten Körnchen für eine ausreichende Zeitdauer;
(v) gegebenenfalls Hinzufügen eines oder mehrerer Zusatzstoffe, Mischen im trockenen Zustand für eine ausreichende Zeit und Passagieren durch ein geeignetes Sieb hindurch;
(vi) gegebenenfalls Hinzufügen von einem oder mehrerer Zusatzstoffen und Mischen im trockenen Zustand für eine ausreichende Zeitdauer;
(vii) Verdichtung der im vorhergehenden Schritt erhaltenen Mischung zu der gewünschten Tablette; und
(viii) gegebenenfalls Ummantelung dieser Tablette.

2. Zusammensetzung nach Anspruch 1, die umfasst:
(a) 5 bis 45 % Wirkstoff;
(b) 25 bis 70 % Polyethylenoxid;
(c) der Rest aus herkömmlichen Zusatzstoffen besteht mit der Ausnahme von basischen Bestandteilen.

3. Zusammensetzung nach Anspruch 1 oder 2, in der der Wirkstoff ein hydrophiler oder lipophiler Wirkstoff ist.

4. Zusammensetzung nach Anspruch 3, in der der Wirkstoff ein hydrophiler Wirkstoff ist.

5. Zusammensetzung nach einem der Ansprüche 1 bis 4, in der der Wirkstoff Aciclovir oder ein pharmazeutisch annehmbares Salz ist.

6. Zusammensetzung nach einem der Ansprüche 1 bis 4, in der der Wirkstoff Nifedipin oder ein pharmazeutisch annehmbares Salz ist.

7. Zusammensetzung nach einem der Ansprüche 1 bis 4, in der der Wirkstoff Nicardipin oder ein pharmazeutisch annehmbares Salz ist.

8. Zusammensetzung nach einem der Ansprüche 1 bis 4, in der der Wirkstoff Captopril oder ein pharmazeutisch annehmbares Salz ist.

9. Zusammensetzung nach einem der Ansprüche 1 bis 4, in der der Wirkstoff Verapamil oder ein pharmazeutisch annehmbares Salz ist.

10. Zusammensetzung nach einem der Ansprüche 1 bis 4, in der der Wirkstoff Diltiazem oder ein pharmazeutisch annehmbares Salz ist.

11. Zusammensetzung nach einem der Ansprüche 1 bis 4, in der der Wirkstoff Oxybutynin oder ein pharmazeutisch annehmbares Salz ist.

12. Zusammensetzung nach einem der Ansprüche 1 bis 4, in der der Wirkstoff Valaciclovir oder ein pharmazeutisch annehmbares Salz ist.

13. Zusammensetzung nach einem der Ansprüche 1 bis 4, in der der Wirkstoff Glipizid oder ein pharmazeutisch annehmbares Salz ist.

14. Zusammensetzung nach einem der Ansprüche 1 bis 4, in der der Wirkstoff Felodipin oder ein pharmazeutisch annehmbares Salz ist.

15. Zusammensetzung nach einem der Ansprüche 1 bis 4, in der der Wirkstoff Isosorbid oder ein pharmazeutisch annehmbares Salz ist.

16. Zusammensetzung nach einem der Ansprüche 1 bis 4, in der der Wirkstoff Carbidopa oder ein pharmazeutisch annehmbares Salz ist.

17. Zusammensetzung nach einem der Ansprüche 1 bis 4, in der der Wirkstoff Levodopa oder ein pharmazeutisch annehmbares Salz ist.

18. Zusammensetzung nach einem der Ansprüche 1 bis 4, in der der Wirkstoff Pentoxiphyllin oder ein pharmazeutisch annehmbares Salz ist.

19. Zusammensetzung nach einem der Ansprüche 1 bis 18, in der das Polyethylenoxid ein Molekulargewicht aufweist, das zwischen 50 000 und 8 000 000, vorzugsweise zwischen 100 000 und 3 000 000 variiert.

20. Zusammensetzung nach einem der Ansprüche 1 bis 19, in der der aus üblichen Zusatzstoffen bestehende Rest mikrokristalline Zellulose, Laktose, Pigmente, Weichmacher, Gleitmittel und andere enthält.

21. Zusammensetzung nach einem der Ansprüche 1 bis 20, in der der aus üblichen Zusatzstoffen bestehende Rest Magnesiumstearat und/oder Glycerinbehenat und/oder Natriumstearylfumarat enthält.

22. Zusammensetzung nach einem der Ansprüche 1 bis 21, die nochmals ummantelt ist.

23. Zusammensetzung nach einem der Ansprüche 1 bis 22, die eine pharmazeutische Zusammensetzung ist.

24. Zusammensetzung nach einem der Ansprüche 1 bis 23 zu ihrer Verwendung als Medikament.

25. Verfahren zur Herstellung einer Zusammensetzung nach einem der Ansprüche 1 bis 24, das die Schritte umfasst:
(i) Mischen im trockenen Zustand eines Wirkstoff, Polyethylenoxid und gegebenenfalls einen oder mehrere Zusatzstoffe für eine ausreichende Zeitdauer;
(ii) gegebenenfalls Hinzufügen von Lösungsmittel, falls ein solches verwendet wird, Mischen für eine ausreichende Zeitdauer;
(iii) Granulierung durch Passagieren durch ein geeignetes Sieb hindurch;
(iv) Trocknen der derartig gebildeten Körnchen über eine ausreichende Zeitdauer;
(v) gegebenenfalls Hinzufügen von einem oder mehreren Zusatzstoffen, Mischen im trockenen Zustand über eine ausreichende Zeit und Passagieren durch ein geeignetes Sieb hindurch;
(vi) gegebenenfalls Hinzufügen von einem oder mehreren Zusatzstoffen und Mischen im trockenen Zustand über eine ausreichende Zeitdauer;
(vii) Verdichtung der im vorhergehenden Schritt erhaltenen Mischung in die gewünschte Tablette; und
(viii) gegebenenfalls Ummantelung dieser Tablette.
26. Verfahren nach Anspruch 25, bei dem das verwendete Lösungsmittel, falls ein solches Lösungsmittel verwendet wird, ein Alkohol ist.
